# EUROPEAN PATENT APPLICATION

(11) **EP 4 538 749 A1**
(43) Date of publication of application: **16.04.2025**
(21) Application number: 23819235.5
(22) Date of filing: 08.06.2023
(51) Int. Cl.: G01T 1/16, G01T 1/20, G01N 23/046, A61B 6/03

(54) **X RAY DETECTOR BASED ON ENERGY INTEGRATING AND PHOTON COUNTING HYBRID IMAGING, AND CT MACHINE**

(30) Priority: 08.06.2022 CN 202210646536
(71) Applicant: Nanovision Technology (Beijing) Co., Ltd., Beijing 100094 (CN)
(72) Inventor: ZHANG, Chao, Beijing 100094 (CN); CUI, Zhili, Beijing 100094 (CN); GAO, Jian, Beijing 100094 (CN)
(74) Representative: Wang, Bo
(86) International application number: PCT/CN2023/099248
(87) International publication number: WO 2023/237071

(57) **Abstract**

Disclosed in the present invention are an X-ray detector based on energy integrating and photon counting hybrid imaging, and a CT machine. The X-ray detector comprises: a counting detector, having at least one column of pixelated electronic elements; and an integrating detector, stacked on one side of the counting detector in a first direction and having at least one column of pixelated photoelectric elements. At any pixel position, the electronic elements and the photoelectric elements are in one-to-one correspondence in the first direction; the counting detector and the integrating detector share one ray conversion part, and the ray conversion part is capable of receiving X-rays and converting the X-rays into electron hole pairs so as to transmit the electron hole pairs to the electronic elements; the ray conversion part is also capable of receiving X-rays and converting the X-rays into visible light photons so as to transmit the visible light photons to the photoelectric elements. The counting detector and the integrating detector in the X-ray detector share one ray conversion part, and image processing can be performed, at a same point in time, on two detection signals at a same pixel position, such that a high-quality reconstructed image is obtained.

## Description

### BACKGROUND

### Technical Field

The present invention relates to the field of computed tomography technologies, relates an X-ray detector based on energy integrating and photon counting hybrid imaging, and also relates to a computed tomography (CT) machine adopting the X-ray detector.

### Related Art

CT is short for computed tomography. The CT uses precisely collimated X-rays and a highly sensitive CT detector to perform profile scanning together around a part of a human body, has characteristics such as fast scanning and a clear image, and may be used in many fields such as medical treatment and security check.

Currently, a commonly used conventional CT detector is an energy integrating detector (EID). This type of energy integrating detector measures an energy integrating signal of an X-ray photon, which may lead to loss of information related to energy, resulting in increased noise and decreased contrast. A photon counting detector is a relatively novel CT detector, has higher conversion efficiency, lower quantum noise, and higher spatial resolution than the conventional energy integrating detector. However, the photon counting detector has relatively weak signal response strength, and has a problem of pulse stacking under high X-ray photon flux.

### SUMMARY

A primary technical problem to be resolved in the present invention is to provide an X-ray detector based on energy integrating and photon counting hybrid imaging.

Another technical problem to be resolved in the present invention is to provide a computed tomography (CT) machine adopting the X-ray detector.

To achieve the above technical objective, the present invention adopts the following s technical solutions:
According to a first aspect of embodiments of the present invention, an X-ray detector based on energy integrating and photon counting hybrid imaging is provided, including:
a counting detector, having at least one column of pixelated electronic elements, to obtain a photon counting image; and
an integrating detector, stacked on a side of the counting detector along a first direction, and having at least one column of pixelated photoelectric elements, to obtain an energy integrating image, where at any pixel position, the electronic elements are in one-to-one correspondence with the photoelectric elements in the first direction.

The counting detector and the integrating detector share a ray conversion portion, the ray conversion portion is configured to receive an X-ray and convert the X-ray into an electron-hole pair, to transmit the electron-hole pair to each of the electronic elements, and the ray conversion portion is further configured to receive the X-ray and convert the X-ray into a visible light photon, to transmit the visible light photon to each of the photoelectric elements.

Preferably, the counting detector further includes:
an electronic circuit board, where the electronic circuit board is provided with an electronic readout circuit in advance.

At least one column of pixelated electronic elements is arranged on a surface of the electronic circuit board close to the integrating detector along a second direction, and any of the electronic elements is connected to the electronic readout circuit.

The second direction is perpendicular to the first direction, and the first direction is perpendicular to the surface of the electronic circuit board.

Preferably, the integrating detector further includes:
a visible light circuit board, where the visible light circuit board is provided with a visible light readout circuit in advance.

At least one column of pixelated photoelectric elements is arranged on a surface of the visible light circuit board close to the electronic circuit board along the second direction, and any of the photoelectric elements is connected to the visible light readout circuit.

Preferably, the ray conversion portion is an integral structure, and is made of a material having both a scintillator property and a semiconductor property.

Preferably, in the first direction, two ends of the ray conversion portion are respectively provided with an electron barrier layer and a hole barrier layer.

Preferably, the ray conversion portion includes:
a first conversion portion, arranged between the electronic circuit board and the visible light circuit board, and made of a semiconductor material, to receive the X-ray and convert the X-ray into the electron-hole pair; and
a second conversion portion, attached to a side of the first conversion portion away from the electronic circuit board, and made of a scintillator material with a hole blocking capability, to receive the X-ray and convert the X-ray into the visible light photon.

Preferably, the counting detector and the integrating detector share a same common circuit board, and the common circuit board is provided with the electronic readout circuit and the visible light readout circuit in advance.

At least one column of pixelated photoelectric elements is arranged on a surface of the common circuit board along a second direction, a pixel position where each of the photoelectric elements is located is provided with an electronic element, any of the photoelectric elements is connected to the visible light readout circuit, and any of the electronic elements is connected to the electronic readout circuit.

The ray conversion portion is arranged on the common circuit board, to receive the X-ray and convert the X-ray into the electron-hole pair and the visible light photon.

Preferably, the counting detector and the integrating detector are wrapped as a whole with a waterproof layer, and the waterproof layer includes at least one or more of a polyimide (PI) film, a polyethylene terephthalate (PET) film, an evaporated parylene film, or a composite coating.

Preferably, the X-ray detector further includes a power supply and a transparent electrode, the transparent electrode is arranged on a side of the ray conversion portion away from the electronic element, one pole of the power supply is connected to the transparent electrode, and the other pole of the power supply is connected to the counting detector, to form an electric field to drive the electron-hole pair.

Preferably, the X-ray detector further includes an image processing portion, the image processing portion is connected to the counting detector, to receive a photon counting image at any pixel position, and the image processing portion is further connected to the integrating detector, to receive an energy integrating image at a same moment and a same pixel position, and performs fusion processing on the photon counting image and the energy integrating image at the same moment and the same pixel position.

According to a second aspect of the embodiments of the present invention, a Ct machine is provided, adopting the above X-ray detector based on energy integrating and photon counting hybrid imaging.

Compared with the prior art, the present invention has the following beneficial effects:
1) The ray conversion portion has both the scintillator property and the semiconductor property, to simultaneously perform conversion of the electron-hole pair and the visible light photon, so that the counting detector and the integrating detector can share a ray conversion portion.
2) At any pixel position, the electronic elements are in one-to-one correspondence with the photoelectric elements in the first direction, so that image processing can be performed on two detection signals at the same moment and the same pixel position, thereby obtaining a high-quality reconstructed image.

### BRIEF DESCRIPTION OF THE DRAWINGS

FIG. 1 is a schematic structural diagram of an X-ray detector based on energy integrating and photon counting hybrid imaging according to a first embodiment of the present invention.
FIG. 2 is a schematic structural diagram of another X-ray detector based on energy integrating and photon counting hybrid imaging according to a first embodiment of the present invention.
FIG. 3 is a schematic structural diagram of an X-ray detector based on energy integrating and photon counting hybrid imaging according to a second embodiment of the present invention.
FIG. 4 is a schematic structural diagram of an X-ray detector based on energy integrating and photon counting hybrid imaging according to a third embodiment of the present invention.
FIG. 5 is an example diagram of a computed tomography (CT) machine adopting the X-ray detector.

### DETAILED DESCRIPTION

Technical content of the present invention is described in detail below with reference to drawings and specific embodiments.

### <First embodiment>

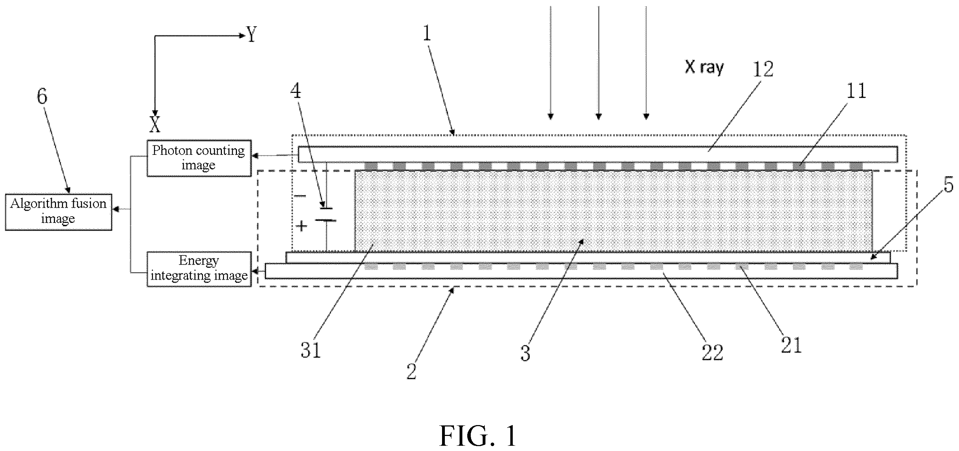

FIG. 1 shows an X-ray detector based on energy integrating and photon counting hybrid imaging according to a first embodiment of the present invention, which includes at least a counting detector 1 and an integrating detector 2. The counting detector 1 has at least one column of pixelated electronic elements 11, to obtain a photon counting image. The integrating detector 2 is stacked on a side of the counting detector 1 along a first direction (that is, a direction X in FIG. 1), and has at least one column of pixelated photoelectric elements 21, to obtain an energy integrating image.

In this embodiment, an electrode is preferably selected as each of the electronic elements 11. In another embodiment, a complementary metal-oxide-semiconductor (CMOS) thin-film transistor or an indium gallium zinc oxide thin-film transistor (IGZO-TFT) may alternatively be selected, to ensure that a surface of each material has an exposed electrode. A photodiode (PD) is preferably selected as each of the photoelectric elements 21. In another embodiment, the PD, a CMOS, a charge-coupled device (CCD), a photomultiplier tube (PMT), or the like may alternatively be selected. At any pixel position, the electronic elements 11 are in one-to-one correspondence with the photoelectric elements 21 in the first direction.

In addition, the counting detector 1 and the integrating detector 2 share a same ray conversion portion 3. The ray conversion portion 3 has both a scintillator property and a semiconductor property, which not only can receive an X-ray and convert the X-ray into an electron-hole pair, to transmit the electron-hole pair to the electronic element 11, but also can receive the X-ray and convert the X-ray into a visible light photon, to transmit the visible light photon to the photoelectric element 21. Therefore, by using X-ray detector based on energy integrating and photon counting hybrid imaging provided in this embodiment, image processing is performed on two detection signals at a same moment and a same pixel position, so that a high-quality reconstructed image can be obtained.

In an embodiment of the present invention, the ray conversion portion 3 has at least one column of pixelated material units 31, and causes, at any pixel position, the electronic elements 11 and the pixelated material units 31 to be in one-to-one correspondence with the photoelectric elements 21 in the first direction. Therefore, correspondence between the ray conversion portion 3 and the counting detector 1 and between the ray conversion portion and the integrating detector 2 can be improved through the pixelated material units 31, to avoid mutual crosstalk.

In the above embodiment, the counting detector 1 may further include an electronic circuit board 12. The electronic circuit board 12 is provided with an electronic readout circuit in advance. The above at least one column of pixelated electronic elements 11 is arranged on a surface (that is, a surface A in FIG. 1) of the electronic circuit board 12 close to the integrating detector 2 along a second direction (that is, a direction Y in FIG. 1), and any of the electronic elements 11 is connected to the electronic readout circuit, so as to be configured to obtain the photon counting image. It may be understood that, in this embodiment, the electronic element 11, the electronic circuit board 12, and the ray conversion portion 3 together form the counting detector 1. As shown in FIG. 1, after the X-ray passes through the electronic circuit board 12 and is irradiated onto the ray conversion portion 3, the X-ray is converted into the electron-hole pair by the ray conversion portion 3. The electron-hole pair is transmitted to the electronic element 11, so that the photon counting image can be obtained through the electronic readout circuit on the electronic circuit board 12.

Similarly, in the above embodiment, the integrating detector 2 may further include a visible light circuit board 22. The visible light circuit board 22 is provided with a visible light readout circuit in advance. The above at least one column of pixelated photoelectric elements 21 is arranged on a surface (that is, a surface B in FIG. 1) of the visible light circuit board 22 close to the electronic circuit board 12 along the second direction, and any of the photoelectric elements 21 is connected to the visible light readout circuit, so as to be configured to obtain the energy integrating image. It may be understood that, in this embodiment, the photoelectric element 21, the visible light circuit board 22, and the ray conversion portion 3 together form the integrating detector 2. As shown in FIG. 1, after the X-ray passes through the electronic circuit board 12 and is irradiated onto the ray conversion portion 3, the X-ray is converted into the visible light photon by the ray conversion portion 3. The visible light photon is transmitted to the photoelectric element 21, so that the energy integrating image can be obtained through the visible light readout circuit on the visible light circuit board 22.

In the above embodiment, the ray conversion portion 3 is an integral structure, and is made of a material having both a scintillator property and a semiconductor property, for example, a lead-based metal halide perovskite material, so that the ray conversion portion 3 can simultaneously convert the electron-hole pair and the visible light photon, to corresponding to the counting detector 1 and the integrating detector 2.

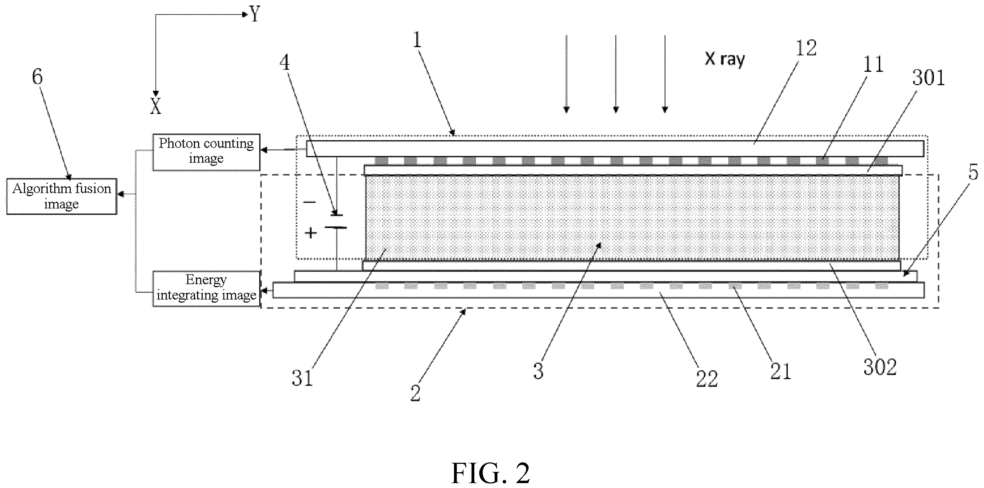

In an embodiment of the present invention, preferably, as shown in FIG. 2, in a first direction (that is, a direction X in FIG. 2), two ends of a ray conversion portion 3 are respectively provided with an electron barrier layer 301 and a hole barrier layer 302. The electron barrier layer 301 is configured to block an electron, and allow a hole to pass smoothly. N-diphenyl-N or N-di (3-methylphenyl) benzidine (poly-TPD) may be selected. Similarly, the hole barrier layer 302 is configured to block the hole, and allow the electron to pass smoothly. PCBM, C60, or a composite coating of perovskite and an organic matter may be selected, with a thickness in a range of 0.1-10 µm. Therefore, flow efficiency of the electron-hole pair may be improved, thereby improving a collection effect of the photon counting image, to facilitate subsequent image reconstruction.

In the above embodiment, the counting detector 1 and the integrating detector 2 are wrapped as a whole with a waterproof layer (not shown). The waterproof layer includes but is not limited to one or more of a polyimide (PI) film, a polyethylene terephthalate (PET) film, an evaporated parylene film, or a composite coating. The composite coating is composed of the PI film, the PET film, and the evaporated parylene film described above in combination with a dense inorganic waterproof film such as an SiO₂ film, a TiO₂ film, or an Al₂O₃ film. Waterproof and moisture-proof performance of the entire X-ray detector based on energy integrating and photon counting hybrid imaging can be improved by using the waterproof layer, thereby improving safety of use.

In the above embodiment, the X-ray detector further includes a power supply 4 and a transparent electrode 5. The transparent electrode 5 is arranged on a side of the ray conversion portion 3 away from the electronic element 11, one pole of the power supply 4 is connected to the transparent electrode 5, and another pole of the power supply 4 is connected to the counting detector 1, to form an electric field to drive the electron-hole pair. A driving force can be provided for movement of the electron-hole pair by using the electric field, to improve movement efficiency of the electron-hole pair.

As shown in FIG. 1, in the above embodiment, the X-ray detector further includes an image processing portion 6. The image processing portion 6 is connected to the counting detector 1, to receive a photon counting image at any pixel position. The image processing portion 6 is further connected to the integrating detector 2, to receive an energy integrating image at a same moment and a same pixel position. Therefore, fusion processing can be performed on the photon counting image and the energy integrating image at the same moment and the same pixel position by using the image processing portion, to obtain a high-quality reconstructed image corresponding to the pixel position.

In summary, according to the X-ray detector based on energy integrating and photon counting hybrid imaging provided in the first embodiment of the present invention, the ray conversion portion 3 has both the scintillator property and the semiconductor property, to perform simultaneous conversion of the electron-hole pair and the visible light photon, so that the counting detector 1 and the integrating detector 2 can share a ray conversion portion. In addition, since at any pixel position, the electronic elements 11 and the material units 31 are in one-to-one correspondence with the photoelectric elements 21 in the first direction, so that image processing can be performed on two detection signals at the same moment and the same pixel position, thereby obtaining the high-quality reconstructed image.

### <Second embodiment>

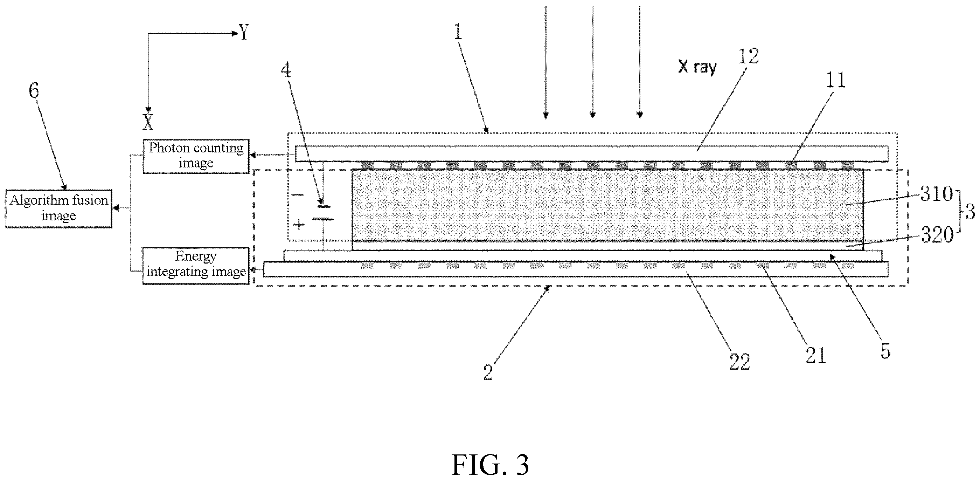

FIG. 3 shows an X-ray detector based on energy integrating and photon counting hybrid imaging according to a second embodiment of the present invention, which includes at least a counting detector 1 and an integrating detector 2. Different from the first embodiment, in this embodiment, a ray conversion portion 3 is a double-layer structure.

Specifically, in this embodiment, the ray conversion portion 3 includes a first conversion portion 310 and a second conversion portion 320. The first conversion portion 310 is made of a semiconductor material, and is arranged between an electronic circuit board 12 and a visible light circuit board 22, to receive an X-ray and convert the X-ray into an electron-hole pair. It may be understood that, in this embodiment, an electronic element 11, the electronic circuit board 12, and the first conversion portion 310 together form the counting detector.

Similarly, the second conversion portion 320 is made of a scintillator material, and is attached to a side of the first conversion portion 310 away from the electronic circuit board 12, to receive the X-ray and convert the X-ray into a visible light photon. It may be understood that, in this embodiment, a photoelectric element 21, the visible light circuit board 22, and the second conversion portion 320 together form the integrating detector.

In an embodiment of the present invention, preferably, when a material of the second conversion portion 320 is selected, the material not only needs to have a scintillator property, but also needs to have a hole blocking capability, for an electron to pass through. More preferably, a side of the first conversion portion 310 away from the second conversion portion 320 is provided with an electron barrier layer, to block the electron from passing through. Therefore, flow efficiency of the electron-hole pair can be improved.

Other than the above differences, other structures of this embodiment are the same as those of the first embodiment, and details are not described again.

### <Third embodiment>

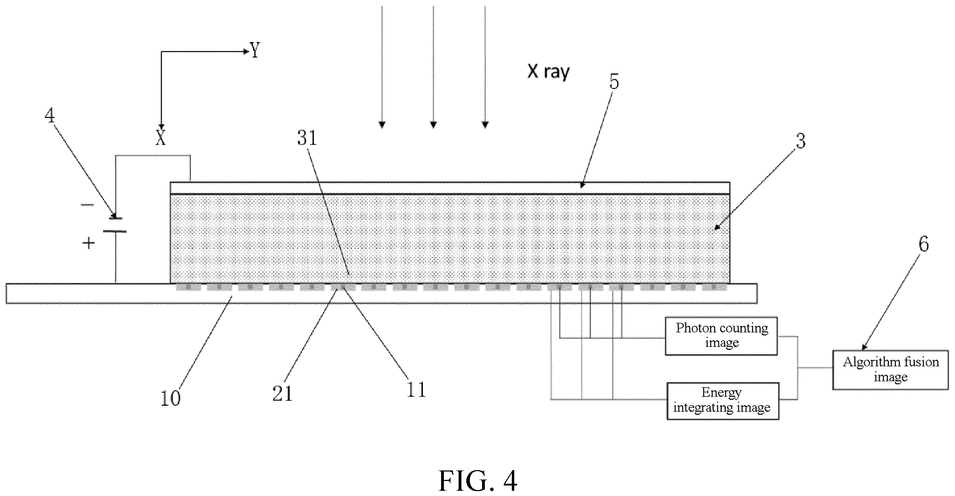

FIG. 4 shows an X-ray detector based on energy integrating and photon counting hybrid imaging according to a third embodiment of the present invention, which includes at least a counting detector 1 and an integrating detector 2. A difference between this embodiment and the first embodiment is that the counting detector and the integrating detector in this embodiment share a same common circuit board 10.

Specifically, in this embodiment, the common circuit board 10 is provided with an electronic readout circuit and a visible light readout circuit in advance. In addition, at least one column of pixelated photoelectric elements 21 is arranged on a surface of the common circuit board 10 along a second direction (that is, a direction Y in FIG. 4), and a pixel position where each of the photoelectric elements 21 is located is provided with an electronic element 11. Any of the photoelectric elements 21 is connected to the visible light readout circuit, and any of the electronic elements 11 is connected to the electronic readout circuit. Correspondingly, a ray conversion portion 3 is arranged on the common circuit board 10, and causes at least one column of pixelated material units 31 to be in one-to-one correspondence with at least one column of pixelated photoelectric elements 21. It should be understood that, in an embodiment of the present invention, the pixelated material units 31 may not be arranged on the ray conversion portion 3, and an overall structure of the ray conversion portion may be formed directly by a material with a scintillator property and a semiconductor property.

It may be understood that, in this embodiment, the electronic element 11, the common circuit board 10, and the ray conversion portion 3 together form the counting detector 1, and the photoelectric element 21, the common circuit board 10, and the ray conversion portion 3 together form the integrating detector 2.

In addition, it may be understood that, since the counting detector 1 and the integrating detector 2 share the same common circuit board 10, a transparent electrode 5 in this embodiment is arranged on a side (that is, a side C in FIG. 4) of the ray conversion portion 3 away from the electronic element 11.

Other than the above differences, other structures of this embodiment are the same as those of the first embodiment, and details are not described again.

It should be noted that the embodiments or variations in the present invention are described in a relevant manner, and same and similar parts between the embodiments or variations may be referred to each other. Each embodiment or variation focuses on a difference from other embodiments. However, the embodiments are implemented based on a working principle of the X-ray detector based on energy integrating and photon counting hybrid imaging, which are not described in detail herein.

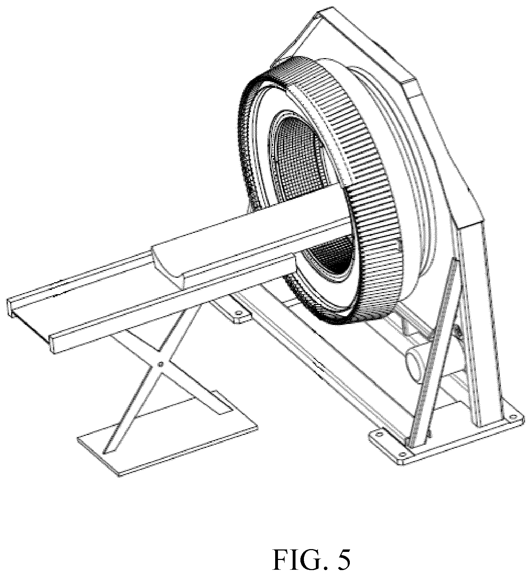

Based on the X-ray detector based on energy integrating and photon counting hybrid imaging provided in the above embodiments, the present invention further provides a computed tomography (CT) machine. As shown in FIG. 5, a CT detector in the Ct machine may be implemented by using the X-ray detector based on energy integrating and photon counting hybrid imaging provided in the present invention. Other assemblies such as a tube and a frame can be implemented by using a conventional design in the prior art, which are described in detail herein.

The X-ray detector based on energy integrating and photon counting hybrid imaging and the corresponding Ct machine provided in the present invention are described in detail above. For a person of ordinary skill in the art, any apparent change made to the present invention without departing from the essential content of the present invention constitutes infringement of the patent right of the present invention and bears a corresponding legal responsibility.

## Claims

1. An X-ray detector based on energy integrating and photon counting hybrid imaging, comprising:
a counting detector, having at least one column of pixelated electronic elements, to obtain a photon counting image; and
an integrating detector, stacked on a side of the counting detector along a first direction, and having at least one column of pixelated photoelectric elements, to obtain an energy integrating image, wherein at any pixel position, the electronic elements are in one-to-one correspondence with the photoelectric elements in the first direction;
the counting detector and the integrating detector share a ray conversion portion, the ray conversion portion is configured to receive an X-ray and convert the X-ray into an electron-hole pair, to transmit the electron-hole pair to each of the electronic elements, and the ray conversion portion is further configured to receive the X-ray and convert the X-ray into a visible light photon, to transmit the visible light photon to each of the photoelectric elements.

2. The X-ray detector based on energy integrating and photon counting hybrid imaging according to claim 1, wherein the counting detector further comprises:
an electronic circuit board, wherein the electronic circuit board is provided with an electronic readout circuit in advance;
at least one column of pixelated electronic elements is arranged on a surface of the electronic circuit board close to the integrating detector along a second direction, and any of the electronic elements is connected to the electronic readout circuit; and
the second direction is perpendicular to the first direction, and the first direction is perpendicular to the surface of the electronic circuit board.

3. The X-ray detector based on energy integrating and photon counting hybrid imaging according to claim 2, wherein the integrating detector further comprises:
a visible light circuit board, wherein the visible light circuit board is provided with a visible light readout circuit in advance; and
at least one column of pixelated photoelectric elements is arranged on a surface of the visible light circuit board close to the electronic circuit board along the second direction, and any of the photoelectric elements is connected to the visible light readout circuit.

4. The X-ray detector based on energy integrating and photon counting hybrid imaging according to claim 3, wherein the ray conversion portion is an integral structure, and is made of a material having both a scintillator property and a semiconductor property.

5. The X-ray detector based on energy integrating and photon counting hybrid imaging according to claim 4, wherein in the first direction, two ends of the ray conversion portion are respectively provided with an electron barrier layer and a hole barrier layer.

6. The X-ray detector based on energy integrating and photon counting hybrid imaging according to claim 3, wherein the ray conversion portion further comprises:
a first conversion portion, arranged between the electronic circuit board and the visible light circuit board, and made of a semiconductor material, to receive the X-ray and convert the X-ray into the electron-hole pair; and
a second conversion portion, attached to a side of the first conversion portion away from the electronic circuit board, and made of a scintillator material with a hole blocking capability, to receive the X-ray and convert the X-ray into the visible light photon.

7. The X-ray detector based on energy integrating and photon counting hybrid imaging according to claim 1, wherein
the counting detector and the integrating detector share a same common circuit board, and the common circuit board is provided with the electronic readout circuit and the visible light readout circuit in advance;
at least one column of pixelated photoelectric elements is arranged on a surface of the common circuit board along a second direction, a pixel position where each of the photoelectric elements is located is provided with an electronic element, any of the photoelectric elements is connected to the visible light readout circuit, and any of the electronic elements is connected to the electronic readout circuit; and
the ray conversion portion is arranged on the common circuit board, to receive the X-ray and convert the X-ray into the electron-hole pair and the visible light photon.

8. The X-ray detector based on energy integrating and photon counting hybrid imaging according to claim 1, wherein the counting detector and the integrating detector are wrapped as a whole with a waterproof layer, and the waterproof layer comprises at least one or more of a polyimide (PI) film, a polyethylene terephthalate (PET) film, an evaporated parylene film, or a composite coating.

9. The X-ray detector based on energy integrating and photon counting hybrid imaging according to claim 1, further comprising a power supply and a transparent electrode, wherein the transparent electrode is arranged on a side of the ray conversion portion away from the electronic element, one pole of the power supply is connected to the transparent electrode, and the other pole of the power supply is connected to the counting detector, to form an electric field to drive the electron-hole pair.

10. The X-ray detector based on energy integrating and photon counting hybrid imaging according to claim 1, further comprising an image processing portion, wherein the image processing portion is connected to the counting detector, to receive a photon counting image at any pixel position, and the image processing portion is further connected to the integrating detector, to receive an energy integrating image at a same moment and a same pixel position, and performs fusion processing on the photon counting image and the energy integrating image at the same moment and the same pixel position.

11. A computed tomography (CT) machine, comprising the X-ray detector based on energy integrating and photon counting hybrid imaging according to any one of claims 1 to 10.
